# EUROPEAN PATENT APPLICATION

(11) **EP 1 319 420 A1**
(43) Date of publication of application: **18.06.2003**
(21) Application number: 01129675.3
(22) Date of filing: 13.12.2001
(51) Int. Cl.: A61M 31/00

(54) **Device for introducing suppositores**

(71) Applicant: Mauro Carmelo, 40050 Villanova di Castenaso (Bologna) (IT)
(72) Inventor: Orlandi, Umberto, 40141 Bologna (IT); Mauro, Carmelo, 40050 Villanova di Castenaso (Bologna) (IT)
(74) Representative: Rinaldi, Carlo

(57) **Abstract**

A device for introducing suppositories consists of a hollow body (1) capable of housing at least a suppository (19), an open end (2) for introducing the suppository (19) into the hollow body (1), a rod or pushing rod (4) entering into the hollow body (1) through an inlet passage, the pushing rod (4) being capable of pushing the suppository (19) towards the end (2); a rounded edge (13) being located near the end (2) for introducing the device into the rectum.

## Description

### BACKGROUND OF THE INVENTION

The present invention relates to a device designed for introducing suppositories; said device comprising a hollow body capable of housing at least a suppository and a rod or a pushing rod capable of pushing the suppository from the hollow body into the rectum.

The applicants do not know instruments capable of carrying out the same function the device, according to the present invention, has been created for.

### AIM AND FEATURES OF THE INVENTION

The aim of the present invention is to remedy these defects. The invention, as claimed, solves the problem of creating a device for introducing suppositories; by means of said device, it is possible to avoid to touch by hands the suppositories to be introduced; in particular, by using the present invention, the suppositories are touched by hands only when they are introduced into the hollow body for the subsequent introduction into the rectum; so doing the hands do not heat the suppositories and do not touch the anal zones.

### BRIEF DESCRIPTION OF THE DRAWINGS

Other advantages, features and aims of the invention, may be more readily understood by referring to the accompanying drawings, which concern preferred embodiments of the device, in which:

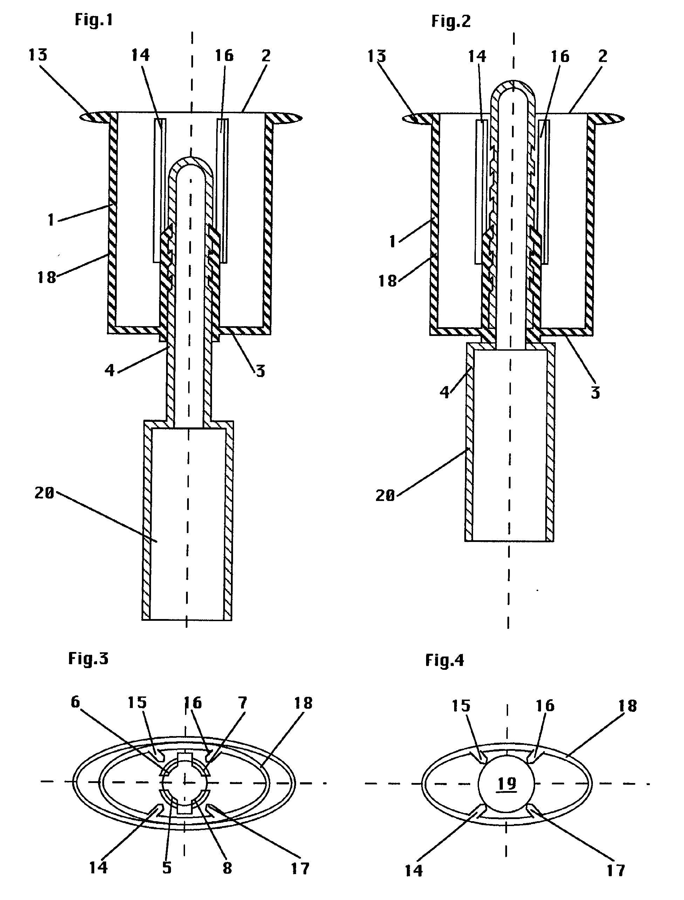

Fig.1 represents a section view of the device with the pushing rod in a first position allowing the introduction of the suppository into the hollow body;
Fig. 2 shows the device of Fig.1 with the pushing rod in a second position allowing the introduction of the suppository into the rectum;
Fig.3 is a view of a section of the device with a plane perpendicular to the axis of symmetry of the device of Fig.1;
Fig.4 is a view of a section of the device with a plane perpendicular to the axis of symmetry of the device of Fig.2;
Figs 5 to 7 schematically represent the functioning of the rod or pushing rod, and
Figs 8, 9 show the functioning of the hollow body capable of housing suppositories of different dimensions.

The scales used for representing the device are useful for illustrating the characterising structures and the functioning of the same device.

### DESCRIPTION OF PREFERRED EMBODIMENTS OF THE INVENTION

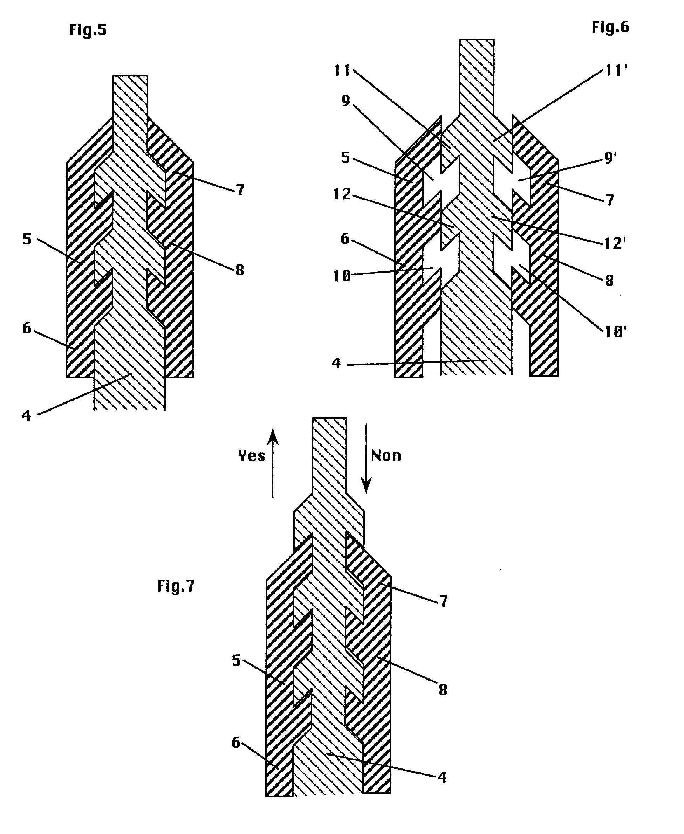

The device consists of a hollow body 1 presenting an open end 2 for introducing a suppository 19 into the hollow body 1; a second end 3 of the hollow body 1 is closed by a wall; a rod or pushing rod 4 enters into the hollow body 1 through a passage consisting of a bushing formed by four sectors 5, 6, 7, 8 of a cylindrical crown elastically pressing the pushing rod 4; every sector 5 ,6, 7, 8 presenting a plurality of female saw-teeth 9, 10 (9' 10') housing further male saw-teeth 11, 12 (11' 12') as shown in detail in Figs 5, 6, 7.

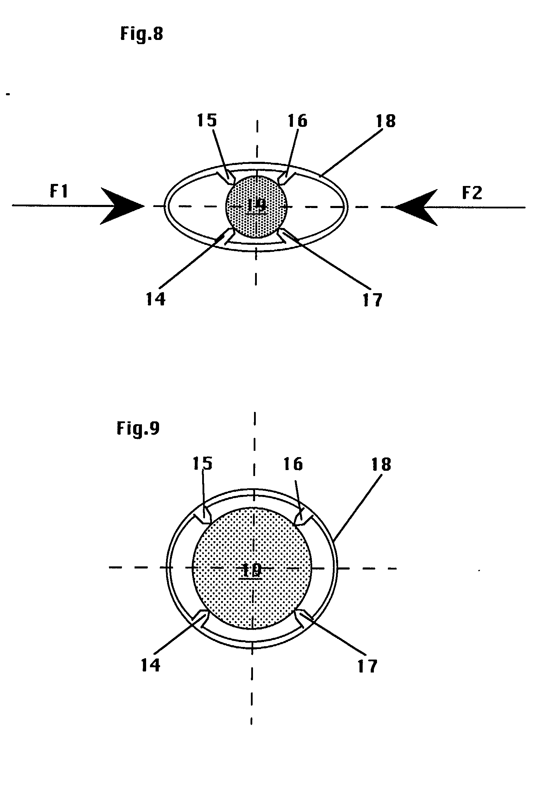

This coupling of the pushing rod 4 with the bushing formed by four sectors 5 ,6, 7, 8 allows the translation of the pushing rod 4 towards the outside of the hollow body 1 only in order to introduce the suppository 19 into the rectum. The opposite translation is always prevented and, in particular, after the introduction of the suppository 19 into the rectum, since the four sectors 5 ,6, 7, 8, elastically opened during the allowed translation of the pushing rod 4, close because of the elasticity of the material so that said sectors 5 ,6, 7, 8 block the pushing rod in the position of Fig.7.

The translation for introducing the pushing rod 4 is carried out by hand by acting on its handle 20; the opposite translation cannot occur; therefore the device can be used just once to solve obvious hygienic problems.

The way of introducing a suppository 19 into the rectum consists in introducing said suppository 19 into the internal part of the hollow body 1, being careful the suppository 19 is parallel to the axis of symmetry of the pushing rod 4 and it has its point faced to the open end 2; the pushing rod 4 allows to eject by hand the suppository 19 from the hollow body 1, therefore, if the open end 2 is inside the rectum, the suppository 19 is introduced into it and it remains in there after the ejection of the device from the rectum, since the pushing rod 4 cannot overtake, in case of opposite translation, the position of Fig.7; in this position the saw-teeth 11, 11' are clamped by the four sectors 5, 6, 7, 8 of the cylindrical crown which tend to increase their resistance to the translation towards the inside of the hollow body with the increasing of the force applied to the pushing rod 4.

In order to allow the introduction of the open end 2 into the rectum a rounded edge 13 is provided near the end 2; this conformation and the elasticity of the material the hollow body 1 is made of allow an easy penetration of the hollow body 1 into the rectum.

The material the hollow body 1 is made of allows as well to house suppositories of any transversal dimension.
As shown in Fig.3, the conformation of the envelope 18 of the hollow body 1 is elliptical in order to house a suppository 19 of small transversal dimension.
For increasing the transversal dimensions, the axes of the ellipse tend to reach the same dimension, until the ellipse becomes a circle housing the suppository 19 having a maximum dimension. The elliptical conformation is changed by the pushing of two opposite forces F1, F2 applied to the envelope 18 by hand.

Finally, four guiding splines 14, 15, 16, 17 are provided inside the envelope 18; said splines 14, 15, 16, 17 developing in parallel to the axis of symmetry of the hollow body 1. The guiding splines 14, 15, 16, 17 define the movement of the suppository 19 inside the envelope 18 in order to maintain a rectilinear translation for keeping the suppository 19 intact.

## Claims

1. Device for introducing suppositories, **characterised by** a hollow body (1) capable of housing at least a suppository (19), an open end (2) for introducing the suppository (19) into the hollow body (1), a rod or pushing rod (4) capable of entering into the hollow body (1) through an inlet passage; the pushing rod (4) being capable of pushing the suppository (19) towards the open end (2); a rounded edge (13) being located near the open end (2) for introducing the device into the rectum.

2. Device as in claim 1, wherein the pushing rod (4) and the inlet passage into the hollow body (1) are fitted with sliding means allowing the translation of the pushing rod (4) towards the open end (2) for introducing the suppository (19) into the rectum, said sliding means preventing the opposite translation in order to make the device useless after the first introduction to solve obvious hygienic problems.

3. Device as in claim 1, wherein the hollow body (1) is delimited by an elliptical envelope (18) which deforms because of the pushing of two opposite forces (F1, F2) applied on the envelope (18) by hand, in order to house suppositories of different dimensions.

4. Device as in claim 1, wherein guiding splines (14, 15, 16, 17) are provided inside the envelope (18); said guiding splines (14, 15, 16, 17) developing in parallel to the axis of symmetry of the hollow body (1).

5. Device as in claims 3, 4, wherein the guiding splines (14, 15, 16, 17) define, for each deformation state of the hollow body (1), the movement of the suppository (19) inside the envelope (18) in order to maintain a rectilinear translation for keeping the suppository (19) intact.
